(19) Europäisches Patentamt
European Patent Office
Office européen des brevets

(11) Veröffentlichungsnummer: **0 269 024**
**A2**

(12) # EUROPÄISCHE PATENTANMELDUNG

(21) Anmeldenummer: 87117165.8

(22) Anmeldetag: 20.11.87

(51) Int. Cl.⁴: **A61K 31/425**

(30) Priorität: 21.11.86 DE 3639888
30.01.87 DE 3702674

(43) Veröffentlichungstag der Anmeldung:
01.06.88 Patentblatt 88/22

(84) Benannte Vertragsstaaten:
AT BE CH DE FR GB IT LI LU NL SE

(71) Anmelder: GÖDECKE AKTIENGESELLSCHAFT
Salzufer 16
D-1000 Berlin 10(DE)

(72) Erfinder: Schmidt, Bernd, Dr.
Hasenbuckweg 14
D-7801 Wittnau(DE)
Erfinder: Laesecke, Klaus, Dr.
Unterdorf 7
D-7800 Freiburg 34(DE)
Erfinder: Weiershausen, Ute
Bundesstrasse 70
D-7803 Gundelfingen(DE)

(74) Vertreter: Mansmann, Ivo
c/o Gödecke AG - Patentabteilung Postfach
569 - Mooswaldallee 1-9
D-7800 Freiburg(DE)

(54) **Verwendung von 3-Methyl-4-oxothiazolidin-2-yliden-acetamid-Derivaten bei der Bekämpfung von AIDS.**

(57) Die Erfindung betrifft die Verwendung von Verbindungen der allgemeinen Formel I

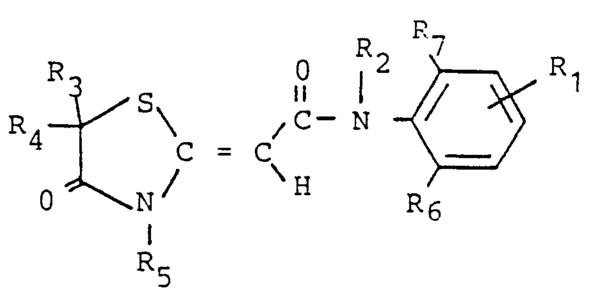

(I)

und insbesondere Ralitolin bei der Bekämpfung von AIDS beim Menschen und bei höheren Säugetieren.

EP 0 269 024 A2

Xerox Copy Centre

## Verwendung von 3-Methyl-4-oxo-thiazolidin-2-ylidenacetamid-Derivaten bei der Bekämpfung von AIDS

AIDS (Acquired Immune Deficiency Syndrome) ist eine sehr ernst zu nehmende Infektionskrankheit, gegen die bisher wirksame Mittel nicht aufgefunden werden konnte.

Verbindungen der allgemeinen Formel I

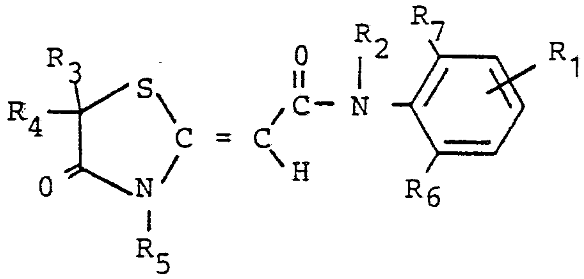

(I)

in welcher die Reste $R_1$ bis $R_7$ gleich oder verschieden sein können und Wasserstoff, ein Chloratom oder einen niederen Alkylrest von 1-4 Kohlenstoffatomen, bevorzugt den Methylrest bedeuten, sind Gegenstand der DE-PS 33 17 000 und stellen wirksame Antiepileptika dar. Besonders Ralitolin (CI-946), (Z)-N-(2-chlor-6-methylphenyl)-(3-methyl-4-oxo-thiazolidin-2-ylidenacetamid der Formel II

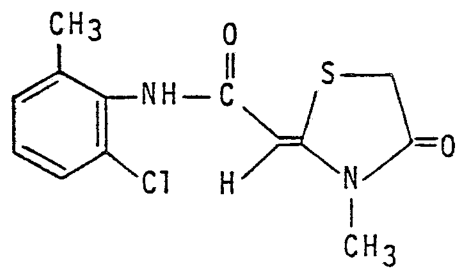

(II)

hat ein breites antikonvulsives Wirkungsprofil bei hervorragender Verträglichkeit.

Bei orientierenden Versuchen mit diesem Wirkstoff hat sich herausgestellt, daß Ralitolin über eine direkte Membranaktion an Lymphozyten deren Rezeptorproteine so verändert, daß das AIDS-Virus sich nicht anlagern kann. Es kann also davon ausgegangen werden, daß sich Ralitolin innerhalb des aus der DE-PS 33 17 000 bekannten verträglichen Dosisbereichs von 10-200 mg pro orale Einzeldosis zur Bekämpfung von AIDS beim Menschen in frühem und fortgeschrittenem Stadium der Krankheit eignet.

Verbindungen der Formel I können beim Menschen in flüssiger oder fester Form enteral oder parenteral appliziert werden. Als Injektionsmedium kommen vor allem wäßrige Phasen zur Anwendung, welche die üblichen Zusätze wie Stabilisierungsmittel und Lösungsvermittler enthalten. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Kalziumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks-und/oder Süßstoffe enthalten.

Gegenstand der vorliegenden Erfindung ist daher die Verwendung von Verbindungen der allgemeinen Formel I zur Herstellung von Arzneimitteln zur Bekämpfung von AIDS.

Besonders bevorzugt sind hierbei die Verbindungen, in welchen entweder $R_1$, $R_2$, $R_3$ und $R_4$ Wasserstoff, $R_5$ und $R_6$ eine Methylgruppe und $R_7$ ein Chloratom darstellt, nämlich Ralitolin, (Z)-N-(2-chlor-6-methylphenyl)-(3-methyl-4-oxo-thiazolidin-2-yliden)-acetamid oder in welchen $R_1$, $R_2$, $R_3$ und $R_4$ Wasserstoff, $R_5$ eine Methylgruppe und $R_6$ und $R_7$ ein Chloratom darstellt, nämlich [(Z)-(3-Methyl-4-oxo-thiazolidin-2-yliden)-N-(2,6-dichlorphenyl) acetamid, Beispiel 2, Variante B der DE-PS 33 17 000].

Bevorzugt sind weiterhin diejenigen Verbindungen der DE-PS 33 17 000, bei denen in der allgemeinen Formel III

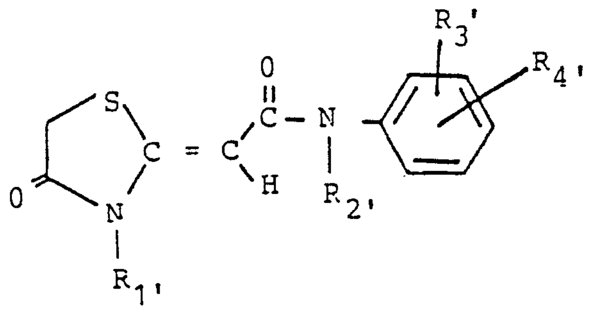

(III)

die Reste $R_1'$ $R_2'$ $R_3'$ und $R_4'$ unabhängig voneinander ein Wasserstoffatom, einen Methyl oder Ethylrest oder ein Chlor-oder Bromatom bedeuten.

Deren Herstellung ist in der DE-PS 33 17 000 beschrieben. Die Herstellung der Verbindungen der allgemeinen Formel I erfolgt in analoger Weise, wie in der DE-PS 33 17 000 für die Verbindungen der allgemeinen Formel III beschrieben.

Ein weiterer Gegenstand der Erfindung sind Arzneimittel, die gegebenenfalls neben den üblichen Zusatz-und Hilfsstoffen Verbindungen der allgemeinen Formel I, insbesondere Ralitolin enthalten zur Behandlung von AIDS.

**Ansprüche**

1.) Verwendung von Verbindungen der allgemeinen Formel I,

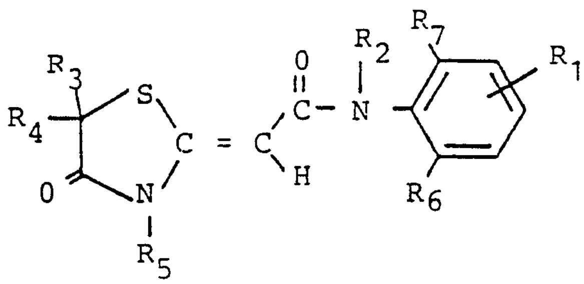

(I)

in welcher die Reste $R_1$ bis $R_7$ gleich oder verschieden sein können und Wasserstoff, ein Halogenatom oder einen niederen Alkylrest von 1-4 Kohlenstoffatomen, bevorzugt den Methylrest bedeuten, zur Herstellung von Arzneimitteln zur Bekämpfung von AIDS beim Menschen und bei höheren Säugetieren.

2.) Verwendung von Verbindungen der allgemeinen Formel III nach Anspruch 1

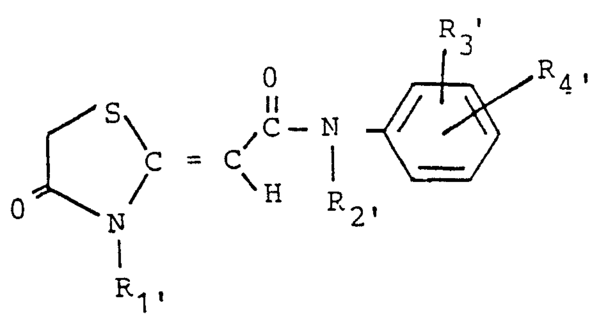

(III)

in welcher die Reste $R_1'$ $R_2'$ $R_3'$ und $R_4'$ unabhängig voneinander ein Wasserstoffatom, einen Methyl oder Ethylrest oder ein Chlor oder Bromatom bedeuten, zur Herstellung von Arzneimitteln zur Bekämpfung von AIDS beim Menschen und den höheren Säugetieren.

3.) Verwendung von (Z)-(3-Methyl-4-oxo-thiazolidin-2-yliden)-N-(2,6-dichlorphenyl) acetamid nach Anspruch 1 und 2 zur Herstellung von Arzneimitteln zur Bekämpfung von AIDS beim Menschen und den höheren Säugetieren.

4.) Verwendung von Ralitolin zur Herstellung von Arzneimitteln zur Bekämpfung von AIDS beim Menschen und bei höheren Säugetieren.

5.) Arzneimittel enthaltend Verbindungen der allgemeinen Formel I gemäß der Ansprüche 1-4 neben Träger-und Hilfsstoffen zur Bekämpfung von AIDS.

6.) Arzneimittel enthaltend Ralitolin neben Träger-und Hilfsstoffen zur Bekämpfung von AIDS.